# EUROPEAN PATENT SPECIFICATION

(11) **EP 2 598 062 B1**
(45) Date of publication and mention of the grant of the patent: **08.02.2017**
(21) Application number: 11813030.1
(22) Date of filing: 26.07.2011
(51) Int. Cl.: A61B 17/70, A61B 17/82, A61F 2/44

(54) **ADJUSTABLE CONNECTOR FOR INTERCONNECTING ELONGATE ROD MEMBERS AT VARIABLE ANGULAR ORIENTATIONS**
EINSTELLBARER KONNEKTOR ZUR VERBINDUNG LÄNGLICHER STANGENELEMENTE IN VERSCHIEDENEN WINKELN
CONNECTEUR RÉGLABLE POUR INTERCONNECTER DES ÉLÉMENTS DE TIGE ALLONGÉS SELON DES ORIENTATIONS ANGULAIRES VARIABLES

(30) Priority: 29.07.2010 US 846298
(43) Date of publication of application: 05.06.2013
(73) Proprietor: Warsaw Orthopedic, Inc., Warsaw, IN 46581 (US)
(72) Inventor: MILLER, Keith, E., Germantown, Tennessee 38138 (US); REZACH, William, Alan, Atoka, Tennessee 38004 (US); SCHWAB, Frank, J., New York, New York 10013 (US)
(74) Representative: Viering, Jentschura & Partner mbB Patent- und Rechtsanwälte
(86) International application number: PCT/US2011/045272
(87) International publication number: WO 2012/015774

(56) References cited:
- KR-A- 20020 036 678
- US-A1- 2007 123 860
- US-A1- 2007 156 142
- US-A1- 2007 156 142
- US-A1- 2007 162 008
- US-A1- 2007 238 335
- US-B1- 6 402 751
- US-B1- 6 872 208
- US-B1- 6 872 208

## Description

### BACKGROUND

Spinal fixation systems are used to adjust, align, stabilize and/or support various portions of the spinal column or other bony structures such as the pelvis, the skull and/or the occiput. One or more elongate spinal rods may be positioned and anchored along the spinal column to provide support and/or to properly position spinal components relative to one another for treatment purposes. Various types of anchors, including bolts, screws and hooks, are commonly used to anchor the elongate spinal rods to the vertebrae.

In some instances, it may be desirable to interconnect two elongate spinal rods together in a side-by-side arrangement at a particular angular orientation such as, for example, when attaching a spinal rod to an existing spinal construct that is already anchored to the spinal column. Various types of connectors have been used to interconnect elongate members to one another. However, prior connector designs have typically been provided with a static configuration wherein the relative angular orientation between the elongate spinal rods is fixed and non-variable. In some instances, it may be desirable to variably position the elongate spinal rods at a select angular orientation, either prior to or during a surgical procedure, due to anatomical features of the patient, the particular physiological problem being treated, and/or the preference of the physician. Although the relative angular orientation between elongate spinal rods may be adjusted via bending or contouring, such techniques are imprecise, rely on the application of significant bending forces, and tend to weaken or degrade the rods.

An adjustable transverse connector is known from, e.g. US 6 872 208 B1. Said transverse connector may be attached to rods of an orthopedic stabilization system. The rods of the stabilization system may be non-parallel and skewed in orientation relative to each other. The transverse connector may include two members that are joined together by a fastener. The transverse connector may be adjustable in three separate ways to allow the transverse connector to attach to the rods. The length of the transverse connector may be adjustable. The rod openings of the transverse connector may be partially rotatable about a longitudinal axis of the transverse connector. Also, a first member may be angled towards a second member so that the transverse connector can be attached to rods that are diverging. The transverse connector may include cam locks that securely attach the transverse connector to the rods. Rotating a cam locks may extend a rod engager into a rod opening. The rod engager may be a portion of the cam lock. The extension of the rod engager into a rod opening may push a rod against a body of the transverse connector to form a frictional engagement between the transverse connector, the rod and the rod engager.

A lateral connector assembly is known from, e.g. US 2007/0238335 A1.

A grommet assembly is known from, e.g. US 2007/0162008 A1.

A Top-tightening side-locking spinal connector assembly is known from, e.g. US 2007/0156142 A1.

Thus, there remains a need for an improved connector for interconnecting elongate rod members at variable angular orientations relative to one another. The present invention satisfies this need and provides other benefits and advantages in a novel and unobvious manner.

### SUMMARY

The invention provides a spinal implant according to claim 1. Further embodiments of the invention are described in the dependent claims.

The present invention relates generally to treatment of the spinal column, and more particularly relates to an adjustable connector for interconnecting elongate rod members at variable angular orientations relative to one another. While the actual nature of the invention covered herein can only be determined with reference to the claims appended hereto, certain forms of the invention that are characteristic of the preferred embodiments disclosed herein are described below.

It is one object of the present invention to provide an adjustable connector for interconnecting elongate rod members at variable angular orientations relative to one another. Further objects, features, advantages, benefits, and aspects of the present invention will become apparent from the drawings and description contained herein.

### BRIEF DESCRIPTION OF THE DRAWINGS

The present invention is illustrated in the figures 1-18 and 20. Figures 19 and 21-23 illustrate examples that are useful for understanding the invention. FIG. 1 is a side perspective view of an adjustable connector according to one form of the present invention:
FIG. 2 is an exploded side perspective view of the adjustable connector shown in FIG. 1.
FIG. 3 is a side view of the adjustable connector shown in FIG. 1.
FIG. 4 is a top view of the adjustable connector shown in FIG. 1.
FIG. 5 is a cross sectional view of the adjustable connector, as taken along line 5-5 of FIG. 4.
FIG. 6 is a side perspective view of a first connector block associated with the adjustable connector shown in FIG. 1.
FIG. 7 is a side view of the first connector block shown in FIG. 6.
FIG. 8 is an end view of the first connector block shown in FIG. 6.
FIG. 9 is a cross sectional view of the first connector block shown in FIG. 6, as taken along line 9-9 of FIG. 8.
FIG. 10 is a side view of a second connector block associated with the adjustable connector shown in FIG. 1.
FIG. 11 is an end view of the second connector block shown in FIG. 10.
FIG. 12 is a cross sectional view of the second connector block, as taken along line 12-12 of FIG. 11.
FIG. 13 is a side perspective view of a first side of a splined washer associated with the adjustable connector shown in FIG. 1.
FIG. 14 is a side perspective view of an opposite second side of the splined washer shown in FIG. 13.
FIG. 15 is a side view of the splined washer shown in FIG. 14.
FIG. 16 is a side view of a retaining clip associated with the adjustable connector shown in FIG. 1.
FIG. 17 is an end view of the retaining clip shown in FIG. 16.
FIG. 18 is a perspective view of the adjustable connector shown in FIG. 1, as engaged with a pair of spinal rods.
FIG. 19 is a perspective view of an adjustable connector according to another form of the present disclosure, as engaged with a pair of spinal rods.
FIG. 20 is a side view of an adjustable connector according to another form of the present invention.
FIG. 21 is a side perspective view of an adjustable connector according to another form of the present disclosure.
FIG. 22 is an exploded side perspective view of the adjustable connector shown in FIG. 21.
FIG. 23 is a cross sectional view of the adjustable connector shown in FIG. 21.

### DESCRIPTION OF THE ILLUSTRATED EMBODIMENTS

For the purposes of promoting an understanding of the principles of the invention, reference will now be made to the embodiments illustrated in the drawings and specific language will be used to describe the same. It will nevertheless be understood that no limitation of the scope of the invention is hereby intended, and that alterations and further modifications to the illustrated devices and/or further applications of the principles of the invention as illustrated herein are contemplated as would normally occur to one skilled in the art to which the invention relates.

In general, the present invention relates to an adjustable connector for interconnecting elongate members at variable angular orientations relative to one another. In a specific embodiment, the present invention is configured to rotatably couple a pair of elongate spinal rods together in a side-by-side manner at variable angular orientations. The adjustable connector includes a first connector member defining a first passage for receiving a first elongate rod, and a second connector member defining a second passage for receiving a second elongate rod, with the first and second connector members interconnected via a swivel or rotor mechanism to permit the connector members and the elongate rods to be rotatably positioned at various angular orientations relative to one another. The adjustable connector further includes a locking mechanism configured to lock the connector members (and in turn the elongate rods) at a select angular orientation relative to one another. In one embodiment, each of the connector members includes a threaded transverse bore that intersects the rod-receiving passage, and a set screw threaded into the transverse bore and into engagement with the elongate rod to securely capture the elongate rod within the rod-receiving passage. In another embodiment, each of the connector members may be provided with a pair of threaded transverse bores that intersect the rod-receiving passage, with a pair of set screws threaded into the transverse bores and into engagement with the elongate rod to securely capture the elongate rod within the rod-receiving passage.

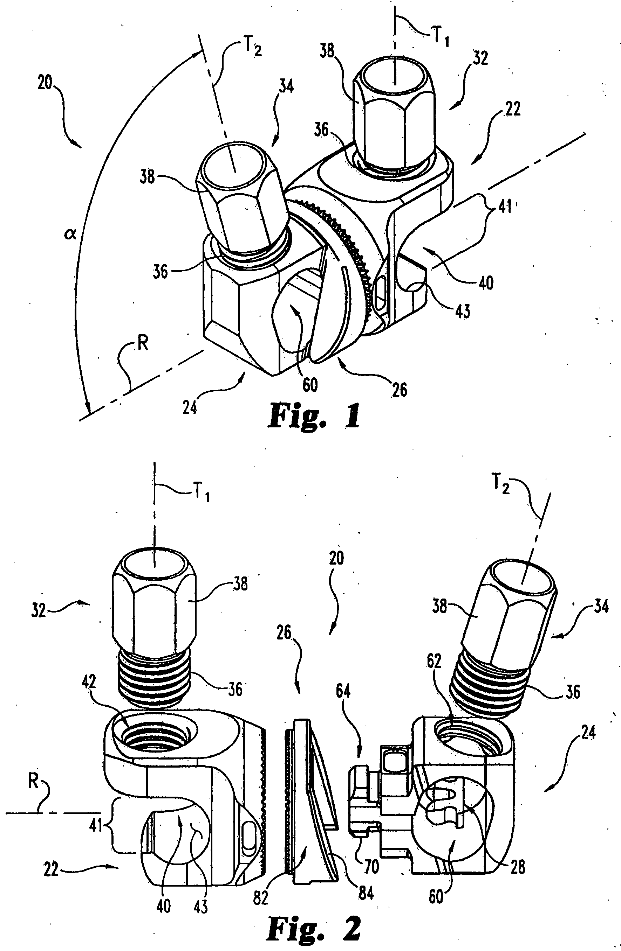

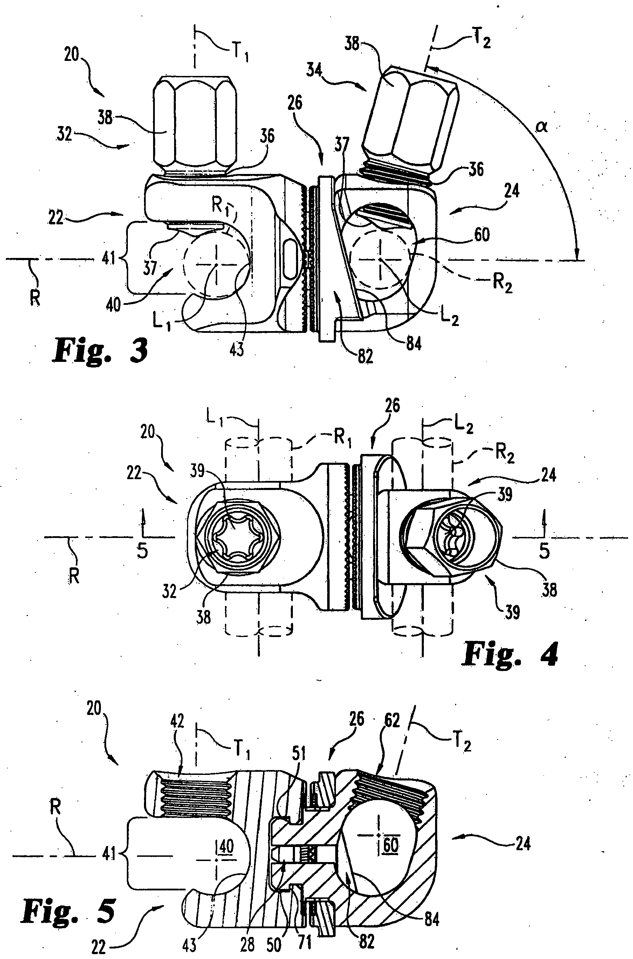

Referring to FIGS. 1-5, shown therein is an adjustable connector 20 according to one form of the present invention for interconnecting elongate rod members R₁, R₂ (shown in phantom in FIGS. 3 and 4) at variable angular orientations relative to one another. In the illustrated forms of the present invention, the adjustable connector embodiments are illustrated and described as interconnecting elongate spinal rods. However, it should be understood that in other forms of the present invention, one or more of the adjustable connector embodiments may interconnect other types and configurations of spinal implants, including bolts, screws and other components used in association with spinal constructs. It should also be understood that the embodiments of the adjustable connector may be used in fields outside of the spinal field including, for example, in fixation or stabilization systems that are attached to other bony structures such as the pelvis, the skull and/or the occiput.

The illustrated embodiment of the adjustable connector 20 generally includes a first connector member or block 22 configured for coupling with a first spinal rod R₁ extending generally along a first longitudinal axis L₁, and a second connector member or block 24 configured for coupling with a second spinal rod R₂ extending generally along a second longitudinal axis L₂. The illustrated embodiment of the adjustable connector 20 also includes a lock member 26 at least partially positioned between the first and second connector members 22, 24 and configured to aid in selectively preventing relative rotational movement between the first and second connector members 22, 24 about a rotational axis R, and a retainer member 28 configured to maintain rotational engagement between the first and second connector members 22, 24. The illustrated embodiment of the adjustable connector 20 further includes a first compression member 32 extending generally along a transverse axis T₁ and configured to engage the first spinal rod R₁ with the first connector member 22, and a second compression member 34 extending generally along a transverse axis T₂ and configured to engage the second spinal rod R₂ with the second connector member 24.

As will be discussed in greater detail below, the adjustable connector 20 is configured to permit relative rotational movement between the first and second connector members 22, 24 about the rotational axis R to allow variation in the angular orientation of the first spinal rod R₁ relative to the second spinal rod R₂, and more specifically between the longitudinal axes L₁, L₂ of the spinal rods. The adjustable connector 20 is also configured to selectively prevent relative rotational movement between the first and second connector members 22, 24 about the rotational axis R to secure the first and second spinal rods R₁, R₂ at a select angular orientation relative to one another, the details of which will be discussed below.

The first connector member 22 includes a first passage 40 sized and configured to receive the first spinal rod R₁ therein, and the second connector member 24 includes a second passage 60 sized and configured to receive a portion of the second spinal rod R₂ therein. In the illustrated embodiment of the adjustable connector 20, the rod-receiving passage 40 has an open configuration defining a lateral opening 41 sized to laterally receive the spinal rod R₁ therethrough such that the spinal rod R₁ may be laterally or transversely inserted into the passage 40. Specifically, the rod-receiving passage 40 has a U-shaped or C-shaped configuration to allow the spinal rod R₁ to be laterally inserted through the lateral opening 41 and into the rod-receiving passage 40 in a direction transverse or normal to the longitudinal axis L₁ of the spinal rod R₁. In the illustrated embodiment of the connector member 22, the lateral opening 41 opens onto a side portion of the first connector member 22 and is arranged generally along the rotational axis R to permit side loading of the spinal rod R₁ into the rod-receiving passage 40. However, it should be understood that the opening 41 may open in other directions to allow top loading, bottom loading, or angled transverse loading of the spinal rod R₁ into the rod-receiving passage 40. Additionally, in the illustrated embodiment of the adjustable connector 20, the second rod-receiving passage 60 has a closed configuration such that the spinal rod R₂ is axially inserted into the passage 60 in a direction extending generally along the longitudinal axis L₂ of the spinal rod R₂. However, it should be understood that the first rod-receiving passage 40 may alternatively have a closed configuration such that the spinal rod R₁ may be axially inserted into the passage 40. and/or the second rod-receiving passage 60 may alternatively have an open configuration such that the spinal rod R₂ may be laterally or transversely inserted into the passage 60.

Additionally, the first connector member 22 includes an aperture 42 communicating with the rod-receiving passage 40 and extending generally along a transverse axis T₁ for receiving the first compression member 32 therethrough. The aperture 42 is positioned and oriented such that displacement of the compression member 32 through the aperture 42 closes off at least a portion of the lateral opening 41 to capture the spinal rod R₁ within the rod-receiving passage 40. In a specific embodiment, the transverse axis T₁ of the aperture 42 is laterally offset from the longitudinal axis L₁ of the spinal rod R₁ and is oriented substantially perpendicular or normal to the rotational axis R and the longitudinal axis L₁. However, it should be understood that other positions and orientations of the transverse axis T₁ of the aperture 42 are also contemplated, including positions where the transverse axis T₁ intersects the longitudinal axis L₁ and/or where the transverse axis T₁ is oriented at an oblique angle, relative to the rotational axis R and/or the longitudinal axis L₁. The second connector member 24 includes an aperture 62 communicating with the rod-receiving passage 60 and extending generally along a transverse axis T₂ for receiving the second compression member 34 therethrough. In the illustrated embodiment, the aperture 62 and the transverse axis T₂ are oriented at an angle α relative to the rotational axis R. In a specific embodiment, the angle a is approximately 75 degrees. However, other angles α are also contemplated as falling within the scope of the present invention. Additionally, in the illustrated embodiment, the transverse axis T₂ of the aperture 62 may be positioned to intersect the longitudinal axis L₂ of the spinal rod R₂. However, it should be understood that other positions and orientations of the aperture 62 and the transverse axis T₂ are also contemplated, including positions and orientations wherein the transverse axis T₂ is offset from the longitudinal axis L₂ of the spinal rod R₂.

In the illustrated embodiment, the compression members 32, 34 are each configured as a set screw, and the apertures 42, 62 in the first and second connector members 22, 24 are internally threaded so as to threadingly receive the set screws 32, 34 therethrough. In the illustrated embodiment, the set screws 32, 34 each have a break-off configuration, including a threaded body portion 36 and a break-off head portion 38. However, it should be understood that other types and configurations of the compression members or set screws 32, 34 are also contemplated as falling within the scope of the present invention, including non-threaded compression members and set screws that do not include a break-off head portion. Furthermore, although the illustrated embodiment of the adjustable connector 20 utilizes a single set screw for use in association with each of the connector members 22, 24 to secure the spinal rods R₁, R₂ within the rod-receiving passages 40, 60, in other embodiments, two or more set screws may be used in association with each of the connector members 22, 24 to secure the spinal rods R₁, R₂ within the rod-receiving passages 40, 60.

In one embodiment, the threaded body portion 36 of the set screws 32, 34 includes a rod-engaging surface 37 (FIG. 3) configured for engagement with an exterior surface of the spinal rods. Additionally, the break-off head portion 38 of the set screws 32, 34 includes a tool receiving recess 39 (FIG. 4) configured to receive a distal end portion of a driver instrument (not shown). In one embodiment, the tool receiving recess 39 has a Torx-shaped configuration. However, other shapes configurations are also contemplated. In a further embodiment, when the set screws 32, 34 are fully engaged with the spinal rods and the head portion 38 is broken away from the threaded body portion 36, the end of the threaded body portion 36 is preferably positioned substantially flush with an outer surface of the connector member 22, 24 to provide the connector with a lower overall profile.

As should be appreciated, since the set screw 32 and the aperture 42 extend along a transverse axis T₁ that is outwardly offset relative to the longitudinal axis L₁ of the spinal rod R₁, displacement of the set screw 32 through the aperture 42 and into engagement with the spinal rod R₁ will urge the spinal rod R₁ in a lateral direction relative to the transverse axis T₁ to compress the spinal rod R₁ against a rod-engaging surface 43 that partially surrounds the rod-receiving passage 40. Compression of the spinal rod R₁ against the rod-engaging surface 43 secures the spinal rod R₁ within the passage 40 to substantially prevent further axial or rotational movement of the spinal rod R₁ relative to the first connector member 22. In the illustrated embodiment, the rod-engaging surface 43 has a circular configuration defining an inner radius that is substantially equal to the outer radius of the spinal rod R₁. However, other shapes and configurations of the rod-engaging surface 43 are also contemplated, including non-arcuate configurations and configurations wherein the inner radius of at least a portion of the rod-engaging surface 43 is reduced relative to the outer radius of the spinal rod R₁ so as to provide two lines of contact between the rod-engaging surface 43 and the spinal rod R₁.

As should also be appreciated, since the set screw 34 and the aperture 62 extend along a transverse axis T₂ that is generally aligned with the longitudinal axis L₂ of the spinal rod R₂, displacement of the set screw 34 through the aperture 62 and into engagement with the spinal rod R₂ will urge the spinal rod R₂ in a direction generally along the transverse axis T₂ to compress the spinal rod R₂ against an engagement surface defined by the lock member 26, the details of which will be set forth below. Compression of the spinal rod R₂ against the lock member 26 secures the spinal rod R₂ within the rod-receiving passage 60 to substantially prevent further axial or rotational movement of the spinal rod R₂ relative to the second connector member 24. Additionally, as will also be discussed below, compression of the spinal rod R₂ against the lock member 26 also serves to lock the connector members 22, 24 and the spinal rods R₁, R₂ at a select angular orientation relative to one another.

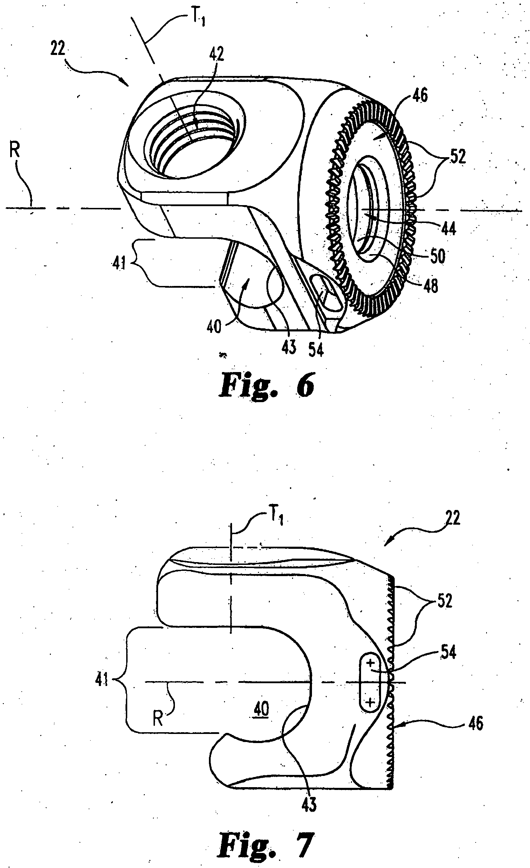

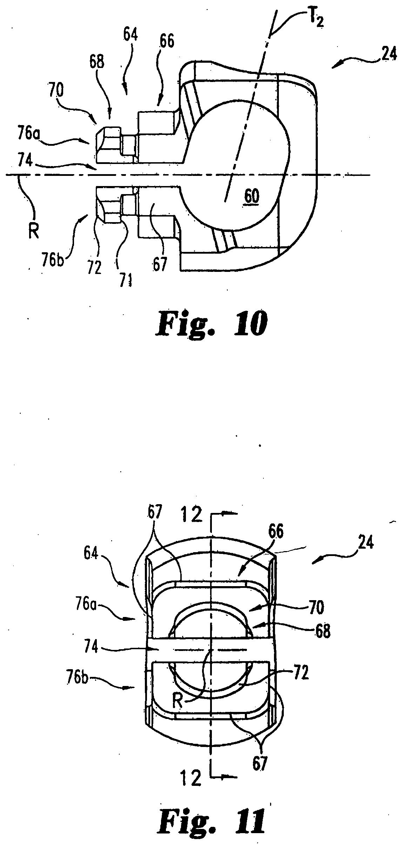

Referring now to FIGS. 6-9, shown therein are additional details regarding the first connector member 22. As indicated above, the first connector member 22 includes a rod-receiving passage 40 defining a lateral opening 41, a threaded aperture 42 in communication with the rod-receiving passage 40 and extending generally along a transverse axis T₁, and a rod-engaging surface 43 at least partially surrounding the passage 40. Additionally, the first connector member 22 includes an opening or recess 44 extending from an outer interface surface 46 and arranged generally along the rotational axis R. As will be discussed in greater detail below, the axial opening 44 is sized and configured to receive a shaft or stem portion 64 extending from the second connector member 24 (FIGS. 10-12) and arranged generally along the rotational axis R. The axial opening 44 and the axially-extending shaft 64 cooperate with one another to permit rotation of the first connector member 22 relative to the second connector member 24 about the rotational axis R. Although the illustrated embodiment of the adjustable connector 20 provides the first connector member 22 with the axial opening 44 and the second connector member 24 with the axial shaft 64, it should be understood that in other embodiments, the first connector member 22 may be provided with the axial shaft 64 and the second connector member 24 may be provided with the axial opening 44. As will be discussed in detail below, the adjustable connector 20 is configured such that the axial shaft 64 is provisionally and positively captured within the axial opening 44 to maintain engagement between the first and second connector members 22, 24 prior to positioning of the first and second spinal rods R₁, R₂ within the first and second rod-receiving passages 40, 60, while still allowing relative rotation between the connector members 22,24 about the rotational axis R.

In one embodiment, the axial opening 44 in the first connector member 22 has a circular configuration and includes an outwardly tapering portion 48 opening onto the outer interface surface 46 of the first connector member 22 to facilitate insertion of the shaft portion 64 of the second connector member 24 into the axial opening 44. The axial opening 44 further includes an enlarged cross sectional portion or undercut region 50 that may be provided in the form of an annular groove extending about the rotational axis R, the purpose of which will be discussed below. Additionally, the axially-facing interface surface 46 of the first connector member 22 includes interengagement structures 52. In one embodiment, the interengagement structures 52 comprise a number of radially-extending splines and grooves positioned about the axial opening 44. In the illustrated embodiment, the radially-extending splines and grooves 52 are positioned entirely about the axial opening 44. However, it should be understood that the radially-extending splines and grooves 52 may instead be positioned about only a portion of the axial opening 44. Additionally, although the illustrated embodiment of the invention depicts the interengagement structures 52 as radially-extending splines and grooves, it should be understood that other types and configurations of interengagement structures are also contemplated for use in association with the present invention. The first connector member 22 may also be provided with tool receiving grooves or recesses 54 defined along opposite sides of the connector member 22 sized and configured for receipt of end portions of an insertion tool or manipulation instrument (not shown).

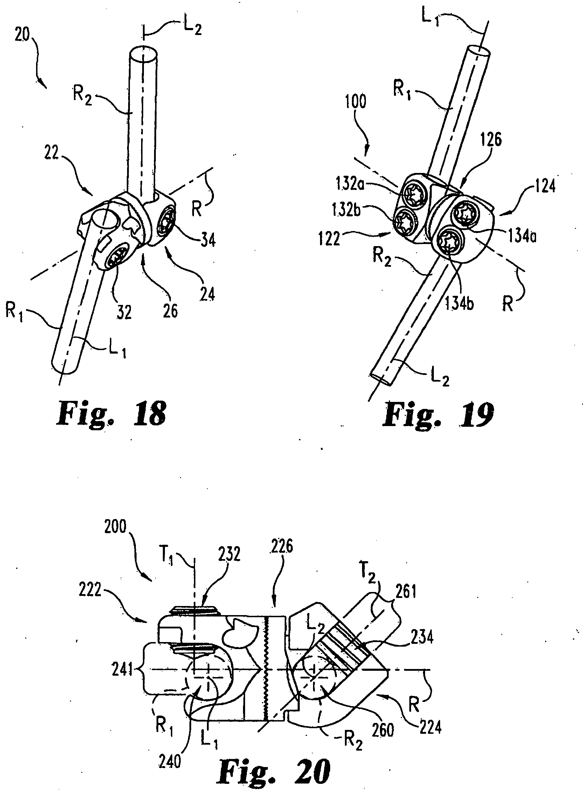

Referring now to FIGS. 10-12, shown therein are additional details regarding the second connector member 24. As indicated above, the second connector member 24 includes a closed rod-receiving passage 60 and a threaded aperture 62 in communication with the rod-receiving passage 60 and extending generally along the transverse axis T₂. However, it should be understood that in other embodiments, the rod-receiving passage 60 may be open to allow for top loading, side loading or bottom loading of the rod into the passage. One such embodiment is illustrated in FIG. 20 and described in detail below.

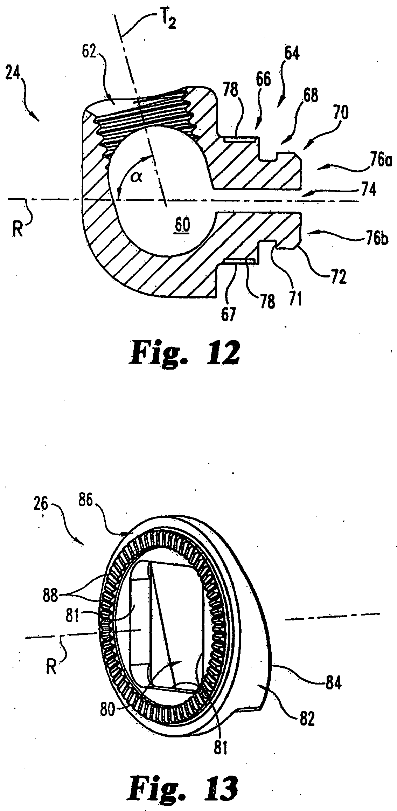

As illustrated in FIGS. 10 and 12, the rod-receiving passage 60 has a slot-like configuration that is elongated and has a length extending generally along the transverse axis T₂. Additionally, the second connector member 24 includes a shaft or stem portion 64 extending generally along the rotational axis R and sized and configured for rotational engagement within the axial opening 44 in the first connector member 22 to permit rotation of the first connector member 22 relative to the second connector member 24 about the rotational axis R. The axial shaft 64 of the second connector member 24 includes a base portion 66 and an axially-extending stem portion 68 having an enlarged peripheral portion 70. The enlarged peripheral portion 70 is sized and shaped for positioning within the undercut region 50 of the axial opening 44 in the first connector member 22 to provisionally and positively capture the axial shaft 64 within the axial opening 44 to maintain engagement between the first and second connector members 22, 24. In the illustrated embodiment, the enlarged peripheral portion 70 of the axial shaft 64 comprises an annular flange located adjacent the distal end of the axial shaft 64 for positioning within the undercut region 50. The enlarged annular flange 70 may define an angled distal end surface or chamfer 72 to facilitate insertion of the enlarged annular flange 70 into the axial opening 44. In the illustrated embodiment, the base portion 66 of the axial shaft 64 has a generally rectangular configuration including flattened or truncated surfaces 67, and the axial stem portion 68 has a generally circular outer cross section. However, it should be understood that other shapes and configurations of the base portion 66 and the axial stem portion 68 are also contemplated.

In one embodiment of the invention, the axial shaft 64 is transitionable from an initial configuration to a reduced transverse profile to allow axial passage of the enlarged annular flange 70 through the axial opening 44 to a position adjacent the undercut region 50 of the first connector member 22. The shaft portion 64 is then expanded back toward the original configuration such that the enlarged annular flange 70 is outwardly expanded into the undercut region 50 of the axial opening 44 to provisionally and positively capture the axial shaft 64 within the axial opening 44. In a specific embodiment, the axial shaft 64 has an elastically resilient configuration to allow the axial shaft 64 to be elastically and resiliently compressed to the reduced transverse profile to allow axial insertion into the axial opening 44, and to then allow the enlarged annular flange 70 to be resiliently expanded into the undercut region 50.

In a more specific embodiment, the axial shaft 64 includes an axial slot 74 extending from the distal end of the axial shaft 64 to the rod-receiving passage 60 to provide the shaft portion 64 with at least two elastically resilient leg portions 76a, 76b that are elastically compressed together to define the reduced transverse profile of the axial shaft 64 and which are resiliently expanded apart such that the enlarged flange 70 is positioned within the undercut region 50 of the axial opening 44. In this manner, the axial shaft 64 is provisionally and positively captured within the axial opening 44 to maintain engagement between the first and second connector members 22, 24 prior to positioning of the first and second spinal rods rod R₁, R₂ within the first and second rod-receiving passages 40, 60. The second connector member 24 may also be provided with grooves or recesses 78 (FIG. 12) defined along opposite sides of the base portion 66 of the axial shaft 64 for receipt of a material or substance such as, for example, a silicone adhesive material (not shown) and/or for receipt of end portions of an insertion tool or manipulation instrument (not shown).

Referring now to FIGS. 13-15, shown therein are additional details regarding the lock member 26. As indicated above, the lock member 26 is configured to aid in selectively preventing relative rotational movement therebetween about the rotational axis R. In one embodiment of the invention, the lock member 26 has disc or washer-like configuration and is at least partially positioned between the first and second connector members 22, 24. Additionally, the lock member 26 defines an opening 80 extending generally along the rotational axis R and sized and shaped to receive the base portion 66 of the axial shaft 64 of the second connector member 24 therethrough. In the illustrated embodiment, the opening 80 has a shape that corresponds to that of the base portion 66 of the axial shaft 64. In one embodiment, the opening 80 has a generally rectangular configuration including flattened or planar surfaces 81. However, it should be understood that other shapes and configurations of the opening 80 are also contemplated. Additionally, it should be appreciated that positioning of the rectangular-shaped base portion 66 of the axial shaft 64 within the rectangular-shaped opening 80 substantially prevents relative rotation between the lock member 26 and the second connector member 24.

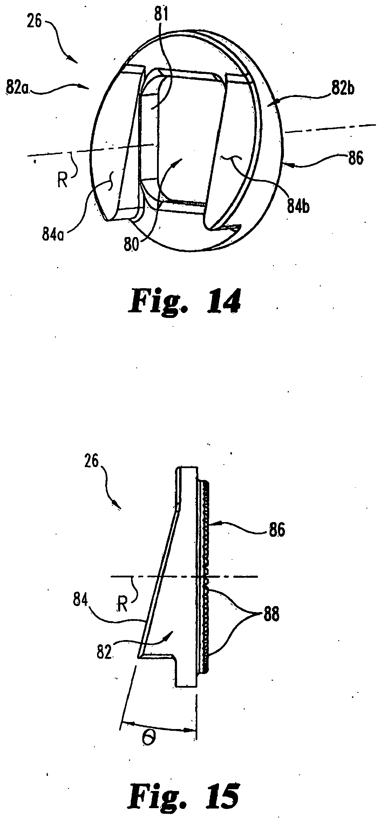

In the illustrated embodiment, the lock member 26 includes at least one axially-extending projection portion 82 defining a rod bearing surface 84 facing a direction along the rotational axis R and intersecting and overlapping a portion of the rod-receiving passage 60 of the second connector member 24. (FIGS. 1 and 4). The lock member 26 further includes an opposite axially-facing interface surface 86 facing the axially-facing interface surface 46 of the first connector member 22. As shown in FIG. 14, the lock member 26 may be provided with a pair of axially-extending projections 82a, 82b arranged on opposite sides of the rotational axis R which define a pair of rod bearing surface 84a, 84b. In the illustrated embodiment, the rod bearing surface 84, 84a, 84b is substantially flat and planar, with the flat/planar rod bearing surface extending from an upper portion of the lock member 26 to a lower portion of the lock member 26 and across the rotational axis R. (FIGS. 3, 14 and 14). In one embodiment, the flat/planar rod bearing surface 84, 84a, 84b is generally symmetrical relative to the rotational axis R. However, other embodiments are also contemplated. Additionally, as shown in FIGS. 1 and 4, when the lock member 26 is assembled with the second connector member 24, the rod bearing surfaces 84a, 84b are arranged on either side of the rod-receiving passage 60 and intersect and overlap a portion of the rod-receiving passage 60, the purpose of which will be discussed below. In a further embodiment, the rod bearing surfaces 84a, 84b each have an angled configuration defining an angle θ relative to the axially-facing interface surface 86. In the illustrated embodiment, the rod bearing surfaces 84a, 84b each define an angle θ relative to the axially-facing interface surface 86 of approximately 14 degrees. However, other angles θ are also contemplated as falling within the scope of the present invention.

In the illustrated embodiment, the axially-facing interface surface 86 of the lock member 26 includes interengagement structures 88. In one embodiment, the interengagement structures 88 comprise a number of radially-extending splines and grooves positioned about the opening 80. In the illustrated embodiment, the radially-extending splines and grooves 88 are positioned entirely about the opening 80. However, it should be understood that the radially-extending splines and grooves 88 may instead be positioned about only a portion of the opening 80. Additionally, although the illustrated embodiment of the invention depicts the interengagement structures 88 as radially-extending splines and grooves, it should be understood that other configurations of interengagement structures are also contemplated for use in association with the present invention. As shown in FIGS. 1 and 4, the interface surface 86 of the lock member 26 is positioned in oppositely-facing relation relative to the interface surface 46 of the first connector member 22. Additionally, as will be discussed below, the radially-extending splines and grooves 88 of the lock member 26 may be selectively interengaged with the radially-extending splines and grooves 52 of the first connector member 22 in an interdigitating manner to selectively prevent relative rotation between the lock member 26 and the first connect member 22, which in turn prevents relative rotational movement between the first and second connector members 22, 24 about the rotational axis R.

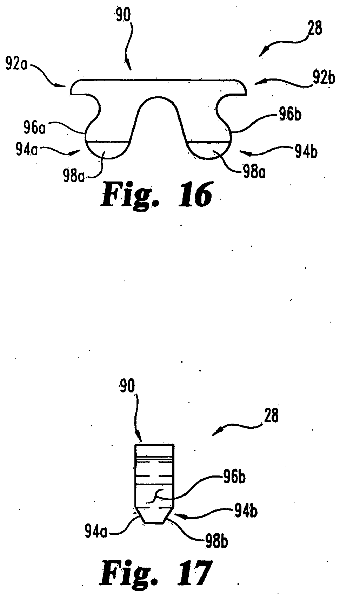

Referring now to FIGS. 16 and 17, shown therein are additional details regarding the retainer member 28. As indicated above, the retainer member 28 is engaged with the first and second connector members 22, 24 and is configured to maintain rotational engagement between the first and second connector members 22, 24. In the illustrated embodiment of the invention, the retainer member 28 is configured as a clip having a spring-like configuration and including a base portion 90 having opposite end portions 92a, 92b, and projecting portions or lobes 94a, 94b extending from the base portion 90 and defining rounded end surfaces 96a, 96b and tapered side surfaces 98a, 98b. Although a particular embodiment of the retainer member 28 has been illustrated and described herein, it should be understood that other types and configurations of the retainer member 28 are also contemplated.

As shown in FIG. 2, subsequent to positioning of the axial shaft 64 of the second connector member 24 within the axial opening 44 in the first connector member 22 and expansion of the enlarged flange portions 70 within the undercut region 50, the retainer member 28 is inserted into the axial slot 74 defined by the axial shaft 64 of the second connector member 24 in a direction generally along the rotational axis R. As the retainer member 28 is axially displaced along the slot 74, the rounded outer surfaces 96a, 96b of the lobes 94a, 94b are compressed against the tapered end surfaces 48 surrounding the axial passage 44 in the first connector member 22, which in turn results in the lobes 94a, 94b being elastically and resiliently compressed toward one another to a reduced transverse profile to allow passage of the lobes 94a, 94b into and through the axial opening 44 to a position adjacent the undercut region 50.

Once the lobes 94a, 94b are positioned adjacent the undercut region 50, the lobes 94a, 94b are resiliently expanded back toward their original configuration wherein the lobes 94a, 94b are outwardly expanded into the undercut region 50 to securely engage the retainer member 28 with the first connector member 22. In a specific embodiment, the retainer member 28 has a spring-like configuration to allow the lobes 94a, 94b to be elastically and resiliently compressed to the reduced transverse profile to allow axial insertion into the axial opening 44, and to then allow the lobes 94a, 94b to be resiliently expanded into the undercut region 50. Once properly engaged with the first connector member 22, the retainer member 28 prevents the elastically resilient leg portions 76a, 76b of the axial shaft 64 from being compressed toward one another to a reduced profile configuration, thereby maintaining rotational engagement of the axial shaft 64 of the second connector member 24 within the axial opening 44 of the first connector member 22. However, it should be understood that the retainer member 28 still allows relative rotational movement between the connector members 22, 24 about the rotational axis R.

Having described the various elements and features associated with the adjustable connector 20, reference will now be made to use and operation of the adjustable connector 20 according to one embodiment of the present invention. As indicated above, the adjustable connector 20 is configured such that the first and second connector members 22, 24 are provisionally engaged to one another in a manner that allows relative rotational movement between the first and second connector members 22, 24 about the rotation axis R. As also indicated above, the spinal rod R₁ may be laterally inserted into the rod-receiving passage 40 of the first connector member 22 via the lateral openings 41, and the spinal rod R₂ may be axially inserted into the rod-receiving passage 60 of the second connector member 24. Once the spinal rods R₁, R₂ are inserted into the rod-receiving passages 40, 60, the sets screws 32, 34 are threaded into the threaded apertures 42 and 62, respectively, in the first and second connector members 22, 24.

As shown in FIG. 3, threading of the set screw 32 through the aperture 42 at least partially closes off the lateral opening 41 to initially retain the spinal rod R₁ within the passage 40. Further displacement of the set screw 32 along the transverse axis T₁ urges the spinal rod R₁ in a lateral direction relative to the transverse axis T₁ and into abutment against the rod-engaging surface 43 of the passage 40. Compression of the spinal rod R₁ against the rod-engaging surface 43 secures the spinal rod R₁ within the passage 40 to substantially prevent further axial or rotational movement of the spinal rod R₁ relative to the first connector member 22. The set screw 34 is initially threaded into the aperture 62 in the second connector member 24, but initially not into tight engagement with the second spinal rod R₂. As illustrated in FIG. 18, the angular orientation of the first spinal rod R₁ relative to the second spinal rod R₂ may then be variably adjusted to a desired angular orientation via rotation of the first connector member 22 relative to the second connector member 24 about the rotational axis R. It should be understood that adjustment of the angular orientation of the first spinal rod R₁ relative to the second spinal rod R₂ via rotation of the first connector member 22 relative to the second connector member 24 may be accomplished during an operative procedure such as, for example, during an osteotomy procedure, during a spinal stabilization procedure, or during other surgical procedures that would occur to those having ordinary skill in the art.

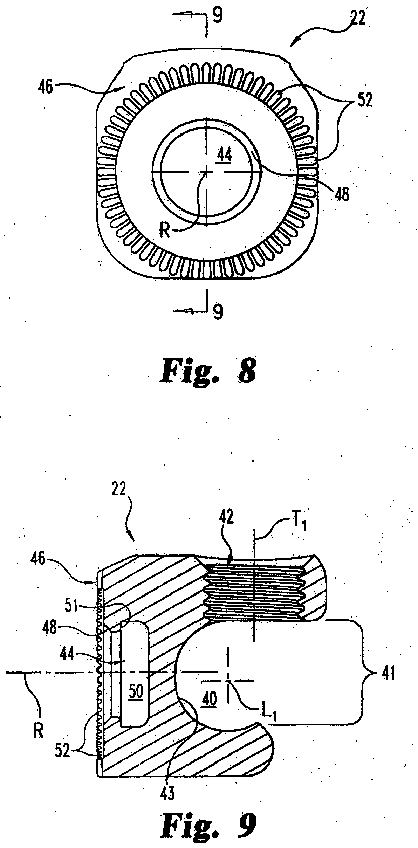

Once the select angular orientation between the first and second spinal rods R₁, R₂ (and the first and second longitudinal axes L₁, L₂) has been achieved, the set screw 34 is driven further into the aperture 62 along the transverse axis T₂ and into compressing engagement with the spinal rod R₂. The set screw 34 urges the spinal rod R₂ into abutting engagement against the rod bearing surfaces 84a, 84b of the lock member 26, which in turn axially urges the lock member 26 toward the first connector member 22 in a direction along the rotation axis R, and which also pulls the first connector member 22 away from the second connector member 24 to compress an axially-facing surface or shoulder 71 defined by the enlarged annular flange 70 of the shaft 72 (FIG. 12) against an opposing axially-facing surface or shoulder 51 defined by the undercut region 50 of the axial opening 44 (FIG. 9).

Displacement of the lock member 26 toward the first connector member 22 compresses the interface surface 86 of the lock member 26 into engagement against the interface surface 46 of the first connector member 22, which in turn results in intermeshing or interdigitating engagement between the radially-extending splines and grooves 88 of lock member 26 and the radially-extending splines and grooves 52 defined by the first connector member 22. Interdigitating or intermeshing engagement between the radially-extending splines and grooves 52, 88 selectively prevents relative rotational movement between the lock member 26 and the first connector member 22. Since the lock member 26 and the second connector member 24 are non-rotatably coupled to one another, selectively preventing relative rotational movement between the lock member 26 and the first connector member 22 correspondingly prevents relative rotational movement between the first and second connector members 22 and 24, thereby locking the spinal rods R₁, R₂ at a select angular orientation relative to one another. Additionally, compression of the spinal rod R₂ between the set screw 34 and the rods bearing surfaces 84a, 84b of the lock member 26 secures the spinal rod R₂ within the rod-receiving passage 60 to substantially prevent further axial or rotational movement of the spinal rod R₂ relative to the second connector member 24. At this point, the spinal rods R₁, R₂ are each secured within the rod-receiving passages 40, 60 of the first and second connector members 22, 24, and the first and second connector members 22, 24 are locked at a select rotational position relative to one another about the rotational axis R, which in turn locks the spinal rods R₁, R₂ at a select angular orientation relative to one another.

In the illustrated embodiment of the adjustable connector 20, the set screw 34 serves two functions. First, the set screw 34 compresses the spinal rod R₂ into abutting engagement against the rod bearing surfaces 84a, 84b of the lock member 26 to lock the spinal rod R₂ within the rod-receiving passage 60 to substantially prevent further axial or rotational movement of the spinal rod R₂ within the passage 60. Second, the set screw 34 axially urges the lock member 26 toward the first connector member 22 (via the spinal rod R₂) to provide intermeshing or interdigitating engagement between the splines 88 of lock member 26 and the splines 52 of the first connector member 22, thereby substantially preventing relative rotational movement between the first and second connector members 22 and 24. However, it should be understood that one or more additional compression members or set screws may be provided to axially engage the lock member 26 with the first connector member 22 to substantially prevent relative rotational movement between the first and second connector members 22 and 24 or other locking or compressive components. For example, a third set screw (not shown) may be provided which is threaded through a passage (not shown) in the first connector member 22 and into secure engagement with the axial shaft 64 of the second connector member 24 to substantially prevent relative rotational movement between the first and second connector members 22 and 24. Additionally, a third set screw may be used to urge the connector members 22, 24 into secure engagement with one another and/or to securely engage other types and configurations of lock members between the connector members 22, 24 to substantially prevent relative rotational movement therebetween.

Referring to FIG. 19, shown therein is an adjustable connector 100 according to yet another form of the present disclosure. In many respects, the adjustable connector 100 is structurally and functionally similar to the adjustable connectors 20 illustrated and described above. Specifically, the adjustable connector 100 generally includes a first connector member or block 122 configured for coupling with a first spinal rod R₁ extending generally along a longitudinal axis L₁, a second connector member or block 124 configured for coupling with second spinal rod R₂ extending generally along a longitudinal axis L₂, a lock member 126 positioned between the first and second connector members 122, 124. However, unlike the adjustable connector 20 which includes a single set screw 32, 34 associated with each of the connector members 22, 24, the adjustable connector 100 includes a first pair of set screws 132a, 132b associated the first connector member 122, and a second pair of set screws 134a, 134b associated the second connector member 124.

As should be appreciated, the adjustable connector 100 is configured to permit relative rotational movement between the first and second connector members 122, 124 about a rotational axis R to allow variation in the angular orientation of the first spinal rod R₁ relative to the second spinal rod R₂, and more specifically between the longitudinal axes L₁, L₂ of the spinal rods. Additionally, unlike the adjustable connector 20 illustrated and described above wherein the first connector member 22 includes a rod receiving passage 40 defining a lateral opening 41 for lateral receipt of the spinal rod R₁, each of the rod-receiving passages defined by the connector members 122, 124 has a closed configuration such that the spinal rods R₁, R₂ are both axially inserted into the rod-receiving passages.

Once the spinal rods R₁, R₂ are inserted into the rod-receiving passages, the first pair of set screws 132a, 132b are tightened to secure the spinal rods R₁ within the rod-receiving passage of the first connector member 122, and the second pair of set screws 134a, 134b are tightened to secure the spinal rods R₂ within the rod-receiving passage of the second connector member 124. Additionally, similar to operation of the adjustable connector 20, tightening of either pair of the set screws 132a, 132b and 134a, 134b may further serve to axially urge the lock member 126 toward the first connector member 122 in a direction along the rotation axis R so as to intermeshingly engage radially-extending splines and grooves associated with the lock member 126 with radially-extending splines and grooves defined by the first connector member 122. Such intermeshing engagement selectively prevents relative rotational movement between the lock member 126 and the first connector member 122, which in turn selectively prevents relative rotational movement between the first and second connector members 122, 124 to thereby lock the spinal rods R₁, R₂ at a select angular orientation relative to one another.

Referring to FIG. 20, shown therein is an adjustable connector 200 according to another form of the present invention. In many respects, the adjustable connector 200 is structurally and functionally similar to the adjustable connector 20 illustrated and described above. Specifically, the adjustable connector 200 generally includes a first connector member or block 222 configured for coupling with a first spinal rod R₁ (shown in phantom), and a second connector member or block 224 configured for coupling with second spinal rod R₂ (shown in phantom). The adjustable connector 200 also includes a lock member 226 at least partially positioned between the first and second connector members 222, 224 and configured to aid in selectively preventing relative rotational movement between the first and second connector members 222, 224 about a rotational axis R, and may also be provided with a retainer member (not shown) configured to maintain rotational engagement between the first and second connector members 222, 224. The adjustable connector 200 also includes a first compression member or set screw 232 extending generally along a transverse axis T₁ and configured to engage the first spinal rod R₁ with the first connector member 222, and a second compression member or set screw 234 extending generally along a transverse axis T₂ and configured to engage the second spinal rod R₂ with the second connector member 224.

The first connector member 222 includes a first passage 240 sized and configured to receive the first spinal rod R₁ therein, and the second connector member 224 includes a second passage 260 sized and configured to receive a portion of the second spinal rod R₂ therein. Additionally, like the adjustable connector 20, the rod-receiving passage 240 defined by the first connector member 222 has an open configuration defining a lateral opening 241 sized to laterally receive the spinal rod R₁ therethrough such that the spinal rod R₁ may be laterally or transversely inserted into the passage 240. However, unlike the adjustable connector 20, the rod-receiving passage 260 defined by the second connector member 224 has an open configuration defining a transverse opening 261 sized to laterally receive the spinal rod R₂ therethrough such that the spinal rod R₂ may be transversely inserted into the passage 260. Specifically, the rod-receiving passage 260 has a U-shaped configuration and the transverse opening 261 opens in a direction toward the top of the connector member 240 to allow the spinal rod R₂ to be top-loaded into the passage 260 in a direction transverse or normal to the longitudinal axis of the spinal rod R₂. However, it should be understood that the opening 261 may open in other directions to allow side loading, bottom loading, or angled transverse loading of the spinal rod R₂ into the rod-receiving passage 260. Additionally, a first set screw 232 is threaded into a threaded aperture in the connector member 222 to close off at least a portion of the lateral opening 241 to capture the spinal rod R₁ within the passage 240. Additionally, a second set screw 234 is threaded into a threaded aperture in the connector member 224 to entirely close off the transverse opening 261 to capture the spinal rod R₂ within the rod-receiving passage 260.

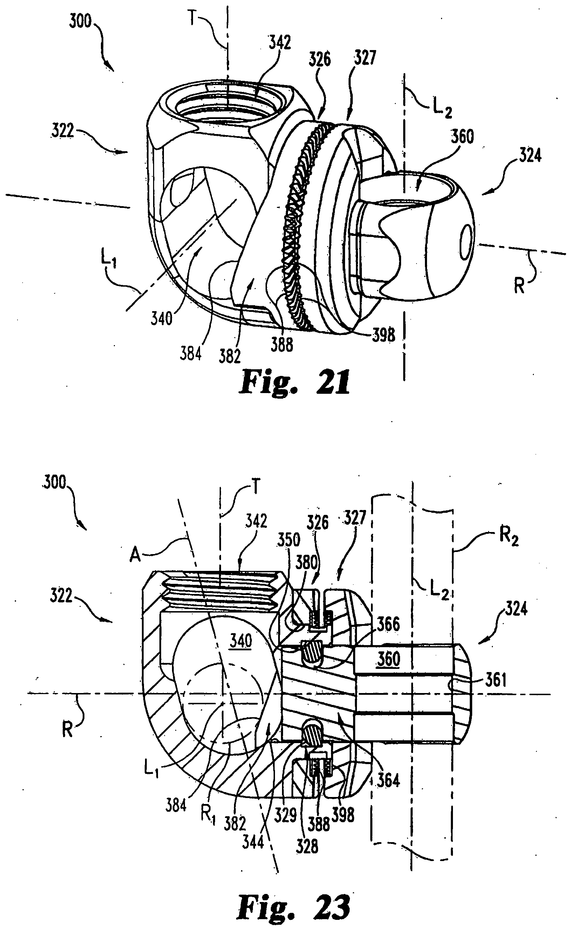

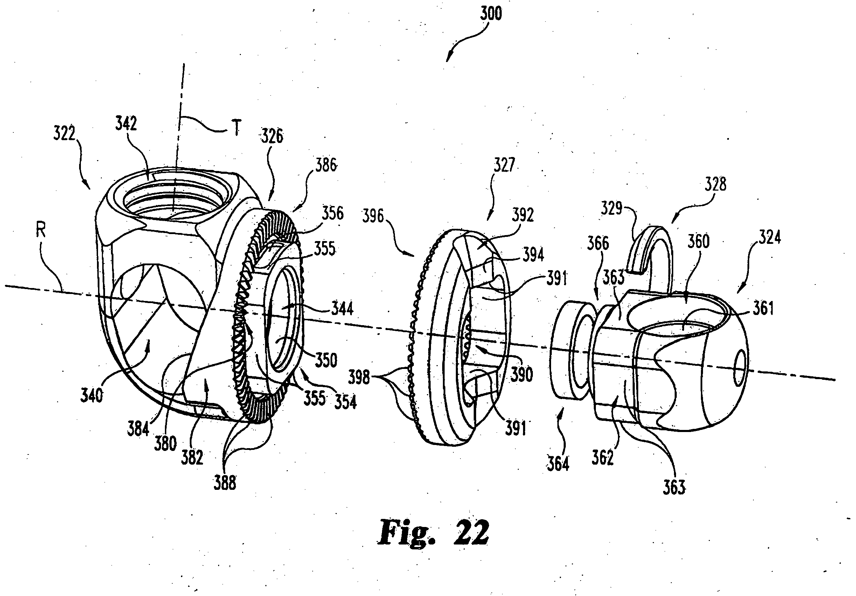

Referring to FIGS. 21-23, shown therein is an adjustable connector 300 according to a further form of the present disclosure for interconnecting elongate rod members R₁, R₂ (shown in phantom in FIG. 23) or other types and configurations of spinal implants at variable angular orientations relative to one another. The adjustable connector 300 generally includes a first connector member or block 322 configured for coupling with a first spinal rod R₁ extending generally along a longitudinal axis L₁, and a second connector member or block 324 configured for coupling with a second spinal rod R₂ extending generally along a longitudinal axis L₂. The adjustable connector 300 also includes a first lock member 326 associated with the first connector member 322 and a second lock member 327 associated with the second connector member 324, and a retainer member 328 engaged between the first and second connector members 322, 324. As will be discussed below, the first and second lock members 326, 327 are configured to aid in selectively preventing relative rotational movement between the first and second connector members 322, 324 about a rotational axis R. As will also be discussed below, the retainer member 328 is configured to maintain rotational engagement between the first and second connector members 322, 324.

Unlike the adjustable connector 20 illustrated and described above, the adjustable connector 300 includes a single compression member (not shown) extending generally along a transverse axis T and configured to engage the first spinal rod R₁ with the first connector member 322. Additionally, the single compression member also engages the second spinal rod R₂ with the second connector member 324, and further engages the first lock member 326 with the second lock member 327 to selectively prevent relative rotational movement between the first and second connector members 322, 324 about a rotational axis R, the details of which will be set forth below. In one embodiment, the single compression member may be configured similar to the set screw 34 illustrated and described above with regard to the adjustable connector 20. However, it should be understood that other types and configurations of compression members are also contemplated.

The first connector member 322 includes a first passage 340 sized and configured to receive the first spinal rod R₁ therein, and the second connector member 324 includes a second passage 360 sized and configured to receive a portion of the second spinal rod R₂ therein. In the illustrated embodiment of the adjustable connector 300, each of the rod-receiving passages 340, 360 has a closed configuration such that the spinal rods R₁, R₂ are axially inserted into the rod-receiving passages 340, 360. However, it should be understood that either or both of the rod-receiving passages 340, 360 may alternatively have an open configuration such that the spinal rods R₁, R₂ may be laterally or transversely inserted into the passages 340, 360. Additionally, the first connector member 322 includes an aperture 342 communicating with the rod-receiving passage 340 and extending generally along a transverse axis T for receiving the compression member therethrough. In the illustrated embodiment, the transverse axis T is arranged substantially normal or perpendicular to the rotational axis R. However, other positions and orientations of the aperture 342 and the transverse axis T are also contemplated. In the illustrated embodiment, the compression member is configured as a set screw, and the aperture 342 in the first connector member 322 is internally threaded so as to threadingly receive the set screw therethrough. However, it should be understood that other types and configurations of the compression member are also contemplated as falling within the scope of the present invention, including a non-threaded compression member.

In the illustrated embodiment, the closed rod-receiving passage 340 of the connector member 322 has an elongate slot-like configuration extending along an axis A that is arranged at an oblique angle relative to both the rotational axis R and the transverse axis T. The first connector member 322 includes an opening or recess 344 arranged generally along the rotational axis R. As will be discussed in greater detail below, the axial opening 344 is sized and configured to receive a shaft or stem portion 364 extending from the second connector member 324 and which is also arranged generally along the rotational axis R. The axial opening 344 and the axial shaft 364 cooperate with one another to permit rotation of the first connector member 322 relative to the second connector member 324 about the rotational axis R. Although the illustrated embodiment of the connector 300 provides the first connector member 322 with the axial opening 344 and the second connector member 324 with the axial shaft 364, it should be understood that in other embodiments, the first connector member 322 may be provided with the axial shaft 364 and the second connector member 324 may be provided with the axial opening 344.

In one embodiment, the axial opening 344 in the first connector member 322 has a circular configuration and includes an enlarged cross sectional portion or undercut region 350 that may be provided in the form of an annular groove, the purpose of which will be discussed below. In the illustrated embodiment of the adjustable connector 300, the first connector member 322 also includes an axial stem portion 354 arranged generally along the rotational axis R, with the axial opening 344 extending through the axial stem portion 354 and into communication with the rod-receiving passage 340. Additionally, the axial stem portion 354 has a generally rectangular configuration including flattened or truncated surfaces 355. However, it should be understood that other shapes and configurations of the axial stem portion 354 are also contemplated. Furthermore, the axial stem portion 354 may be provided with grooves or recesses 356 defined along opposite sides of the axial stem portion 354 for receipt of a material or substance such as, for example, a silicone adhesive material (not shown).

In the illustrated embodiment, the closed rod-receiving passage 360 of the connector member 324 has a circular configuration substantially corresponding to the outer cross section of the spinal rod R₂. Additionally, the rod-receiving passage 360 may be provided with an undercut region or annular groove 361 to provide at least two annular lines of contact between the connector member 324 and the spinal rod R₂ to facilitate more secure engagement with the spinal rod R₂. In the illustrated embodiment, the second connector member 324 includes a base portion 362 and a shaft or stem portion 364 extending therefrom generally along the rotational axis R and sized and configured for rotational engagement within the axial opening 344 in the first connector member 322 to permit rotation of the first connector member 322 relative to the second connector member 324 about the rotational axis R. In the illustrated embodiment, the base portion 362 of the connector member 324 has a generally rectangular configuration including flattened or truncated surfaces 363. However, other shapes and configurations of the base portion 362 are also contemplated. The axial shaft 364 of the second connector member 324 includes an annular groove or recess 366 extending at least partially thereabout. The annular groove 366 is located for positioning adjacent the undercut region 350 of the axial opening 344 in the first connector member 322 when the axial shaft 364 is positioned within the axial opening 344.

In the illustrated embodiment, the retainer member 328 is in the form of an elastically resilient C-shaped clip or snap ring and includes an axially-facing tapered surface 329. As shown in FIG. 23, prior to positioning of the axial shaft 364 of the second connector member 324 within the axial opening 344 in the first connector member 322, the C-shaped clip 328 is positioned within the annular groove 366 of the axial shaft 364. As the shaft 364 is inserted into the axial opening 344 in the first connector member 322, the tapered outer surface 329 of the C-shaped clip 328 is compressed against an axially-facing surface of the stem portion 354, which in turn inwardly and elastically deforms the clip 328 to a reduced outer profile sized for axial insertion into the axial opening 344. Once the C-shaped clip 328 is positioned in alignment with the undercut region 350, the C-shaped clip 328 is allowed to resiliently snap back toward its original configuration wherein the clip 328 is outwardly expanded into the undercut region 350. Positioning of the retainer clip 328 within the annular groove 366 of the axial shaft 364 and the undercut region 350 of the axial opening 344 maintains the first and second connector members 322, 324 in rotational engagement with one another. However, it should be understood that the retainer clip 328 still allows relative rotational movement between the connector members 322, 324 about the rotational axis R. Additionally, although a particular embodiment of the retainer member 328 has been illustrated and described herein, it should be understood that other configurations of the retainer member 328 are also contemplated.

In the illustrated embodiment, the first lock member 326 has disc or washer-like configuration defining an opening 380 extending generally along the rotational axis R and sized and shaped to receive the axial stem portion 354 of the first connector member 322 therethrough. In the illustrated embodiment, the opening 380 has a shape that corresponds to that of the axial stem portion 354. In one embodiment, the opening 380 has a generally rectangular configuration including flattened or truncated surfaces. However, it should be understood that other shapes and configurations of the opening 380 are also contemplated. Additionally, it should be appreciated that positioning of the rectangular-shaped axial stem portion 354 within the rectangular-shaped opening 380 substantially prevents relative rotation between the first lock member 326 and the first connector member 322. The first lock member 326 also includes a pair of axially-extending projections 382 arranged on opposite sides of the rotational axis R and defining a pair of rod bearing surface 384 facing a direction along the rotational axis R and intersecting and overlapping a portion of the rod-receiving passage 340 of the connector member 322. The lock member 326 further includes an opposite axially-facing interface surface 386 which faces an axially-facing interface surface 396 of the second lock member 327. Additionally, the rod bearing surfaces 384 are provided with an angled configuration defining an oblique relative to the axially-facing interface surface 386.

In the illustrated embodiment, the axially-facing interface surface 386 of the first lock member 326 includes interengagement structures 388. In one embodiment, the interengagement structures 388 comprise a number of radially-extending splines and grooves positioned about the rectangular-shaped opening 380. In the illustrated embodiment, the radially-extending splines and grooves 388 are positioned entirely about the opening 380. However, it should be understood that the radially-extending splines and grooves 388 may instead be positioned about only a portion of the opening 380. Additionally, although the illustrated embodiment of the first lock member 326 depicts the interengagement structures 388 as radially-extending splines and grooves, it should be understood that other configurations of interengagement structures are also contemplated for use in association with the present invention.

In the illustrated embodiment, the second lock member 327 also has disc or washer-like configuration defining an opening 390 extending generally along the rotational axis R and sized and shaped to receive the base portion 362 of the second connector member 324 therethrough. In the illustrated embodiment, the opening 390 has a shape that corresponds to that of the base portion 362. In one embodiment, the opening 390 has a generally rectangular configuration including flattened or truncated surfaces 391. However, it should be understood that other shapes and configurations of the opening 390 are also contemplated. Additionally, it should be appreciated that positioning of the rectangular-shaped base portion 362 within the rectangular-shaped opening 390 substantially prevents relative rotation between the second lock member 327 and the second connector member 324. The second lock member 327 also includes a rod-receiving groove 392 arranged on one side of the lock member 327 and generally aligned with the rod-receiving passage 360 of the connector member 324. The second lock member 327 further includes an opposite axially-facing interface surface 396 which faces the axially-facing interface surface 386 of the first lock member 326. Additionally, the rod-receiving groove 392 defines a rod bearing surface 394 for engagement with the spinal rod R₂.

In the illustrated embodiment of the second lock member 327, the axially-facing interface surface 396 of the second lock member 327 includes interengagement structures 398. In one embodiment, the interengagement structures 398 comprise a number of radially-extending splines and grooves positioned about the rectangular-shaped opening 390. In the illustrated embodiment, the radially-extending splines and grooves 398 are positioned entirely about the opening 390. However, it should be understood that the radially-extending splines and grooves 398 may instead be positioned about only a portion of the opening 390. Additionally, as will be discussed below, the radially-extending splines and grooves 388, 398 of the first and second lock members 326, 327 are selectively interengaged with one another in an interdigitating manner to substantially prevent relative rotation between the first and second lock members 326, 327, which in turn substantially prevents relative rotational between the first and second connector members 322, 324.

Having described various elements and features associated with the adjustable connector 300, reference will now be made to use and operation of the adjustable connector 300 according to one embodiment of the present invention. As indicated above, the adjustable connector 400 is configured such that the first and second connector members 322, 324 are engaged to one another in a manner allowing relative rotational movement between the first and second connector members 322, 324 about the rotation axis R. As also indicated above, the spinal rod R₁ may be axially inserted into the closed rod-receiving passage 340 of the first connector member 322, and the spinal rod R₂ may be axially inserted into the closed rod-receiving passage 360 of the seconde connector member 324. The angular orientation of the first spinal rod R₁ relative to the second spinal rod R₂ is then adjusted to a desired angular orientation via rotation of the first connector member 322 relative to the second connector member 324 about the rotational axis R. Once the select angular orientation between the first and second spinal rods R₁, R₂ has been achieved, the set screw is driven through the aperture 342 along the transverse axis T and into compressing engagement with the spinal rod R₁. The set screw urges the spinal rod R₁ into abutting engagement against the rod bearing surfaces 384 of the first lock member 326, which result in axial displacement of the first lock member, 326 into engagement with the second lock member 327, which in turn axially displaces the second lock member 327 toward the second connector member 324 and into compressing engagement with the second spinal rod R₂.

Threading the set screw through the aperture 342. in the connector members 322 serves multiple functions. First, tightening the set screw against the spinal rod R₁ compresses the spinal rod R₁ into abutting engagement against the rod bearing surfaces 384 of the first lock member 326 to thereby prevent further axial or rotational movement of the spinal rod R₁ with the rod-receiving passage 340. Second, tightening the set screw also compresses the splined interface surface 386 of the first lock member 326 into intermeshing or interdigitating engagement with the splined interface surface 396 of the second lock member 327, which in turn selectively prevents relative rotational movement between the lock members 326 and 327 and relative rotational movement between the first and second connector members 322, 324, thereby locking the spinal rods R₁, R₂ at a select angular orientation relative to one another. Third, tightening the set screw also compresses the rod bearing surface 394 of the second lock member 327 against the spinal rod R₂, which in turn compressingly engages the spinal rod R₂ against an inner rod-engaging surface defined by the rod-receiving passage 360 of the second connector member 324 to substantially prevent further axial or rotational movement of the spinal rod R₂ relative to the second connector member 324. Accordingly, a single compression member is used to secure the spinal rods R₁, R₂ within the rod-receiving passages 340, 360 of the first and second connector members 322, 324, and to lock the first and second connector members 322, 324 at a select rotational position relative to one another about the rotational axis R, which in turn locks the spinal rods R₁, R₂ at a select angular orientation relative to one another.

While the invention has been illustrated and described in detail in the drawings and foregoing description, the same is to be considered as illustrative and not restrictive in character, it being understood that only the preferred embodiments have been shown and described and that all changes and modifications that come within the spirit of the invention are desired to be protected.

## Claims

1. An adjustable connector (20; 200) for interconnecting first and second elongate spinal rods (R₁; R₂) at select angular orientations relative to one another, in combination with the said elongate spinal rods, comprising:
a first rod connector member (24; 224) and a second rod connector member (22; 222), wherein the first rod connector member (24; 224) is rotationally coupled to the second rod connector member (22; 222) to permit relative rotation between said first and second rod connector members about a rotational axis (R), said first rod connector member (24; 224) defining a first passage (60; 260) sized and configured to receive the first spinal rod therein, said first passage (60; 260) has a slot-like configuration having a length extending along a transverse axis (T₂) arranged transverse to said rotational axis (R), said second rod connector member (22; 222) defining a second passage (40; 240) sized and configured to receive the second spinal rod therein;
a lock member (26; 226) positioned at least partially between said first and second rod connector members (24, 22; 224, 222), said lock member (26-; 226) including an angled rod bearing surface (84) and an opposite first interface surface (86), said angled rod bearing surface extending along a plane oriented at an oblique angle relative to said rotational axis (R); and
a compression member (34) extending into said first passage (60; 260) of said first rod connector member (24; 224) and into compressed engagement with said first spinal rod to displace said first spinal rod along said transverse axis (T₂) and along said length of said elongated slot and into sliding engagement with said angled rod bearing surface (84) of said lock member (26; 226) to secure said first spinal rod within said first passage (60; 260); and
wherein sliding engagement of said first spinal rod against said rod bearing surface (84) axially displaces said lock member (26; 226) toward said second rod connector member (22; 222) generally along said rotational axis (R) to compress said first interface surface (86) of said lock member (26; 226) against an opposing second interface surface (46) to selectively prevent said relative rotation between said first and second rod connector members (24, 22; 224, 222),
wherein at least one of said first and second rod connector members (24, 22; 224, 222) defines a lateral opening (41; 241) communicating with a corresponding one of said first and second passages (60, 40; 260, 240) to allow one of said first and second spinal rods (R₁, R₂) to be laterally inserted through said lateral opening (41; 241) and into said corresponding one of said first and second passages and
wherein said rotational axis (R) intersects said lateral opening (41; 241).

2. The adjustable connector (20; 200) of claim 1, wherein said transverse axis (T₂) of said elongated slot is arranged at an oblique angle relative to said rotational axis (R).

3. The adjustable connector (20; 200) of claim 2, wherein said elongated slot is bounded by oppositely facing and substantially parallel side surfaces extending along said transverse axis (T₂) at said oblique angle.

4. The adjustable connector (20; 200) of any claims 1-3,
wherein said angled rod bearing surface (84) is substantially flat and planar along said plane.

5. The adjustable connector (20; 200) of claim 4, wherein said angled rod bearing surface (84) is substantially flat and planar from an upper portion of said lock member (26; 226), across said rotational axis (R), and along a lower portion of said lock member.

6. The adjustable connector (20; 200) of any claims 1-5,
wherein said second interface surface (46) is defined by an outer face of said second rod connector member (22; 222).

7. The adjustable connector (20; 200) of claim 6, wherein said lock member (26; 226) comprises a single-piece lock member located between said first spinal rod (R₁; R₂) positioned within said elongated slot in said first rod connector member (24; 224) and said second interface surface (46) defined by said outer face of said second rod connector member (22; 222).

8. The adjustable connector (20; 200) of any claims 1-7, wherein said first and second interface surfaces (86; 46) include a number of radially-extending splines interdigitating with a number of radially-extending grooves to selectively prevent said relative rotation between said first and second rod connector members (24, 22; 224; 222).

9. The adjustable connector (20; 200) of any claims 1-8,
wherein said compression member comprise a first compression member (34) extending into said first passage (60; 260) of said first rod connector member (24; 224) and into compressed engagement with said first spinal rod to secure said first spinal rod within said first passage; and
further comprising a second compression member (32; 232) extending into said second passage (40; 240) of said second rod connector member (22; 222) and into compressed engagement with said second spinal rod to secure said second spinal rod within said second passage.

10. The adjustable connector (20; 200) of claim 9, further comprising a pair of said first compression members extending into said first passage of said first rod connector member (24; 224) and into compressed engagement with said first spinal rod to secure said first spinal rod within said first passage; and
a pair of said second compression members extending into said second passage of said second rod connector member (22; 222) and into compressed engagement with said second spinal rod to secure said second spinal rod within said second passage.

11. The adjustable connector (20; 200) of any claims 1-10,
wherein one of said first and second rod connector members (24, 22; 224; 222) includes an opening (44) extending generally along said rotational axis (R), and wherein the other of said first and second rod connector members includes a shaft (64) rotatably engaged within said opening to permit said relative rotation between said first and second rod connector members about said rotational axis (R), said opening (44) including an undercut region (50) defining an annular groove, said shaft (64) including an enlarged peripheral portion extending transversely therefrom and positioned within said annular groove to positively capture said shaft (64) within said opening (44) to maintain rotational engagement between said first and second rod connector members (24, 22; 224; 222).

## Patentansprüche

1. Einstellbarer Verbinder (20; 200) zum Miteinander-Verbinden einer ersten und einer zweiten langgestreckten Wirbelsäulenstange (R₁; R₂) in ausgewählten Winkelausrichtungen relativ zueinander, in Kombination mit den langgestreckten Wirbelsäulenstangen, aufweisend:
ein erstes Stangenverbinderelement (24; 224) und ein zweites Stangenverbinderelement (22; 222), wobei das erste Stangenverbinderelement (24; 224) drehbar verbunden ist mit dem zweiten Stangenverbinderelement (22; 222), um Relativrotation zwischen dem ersten und dem zweiten Stangenverbinderelement um eine Rotationsachse (R) zu erlauben, wobei das erste Stangenverbinderelement (24; 224;) eine erste Passage (60; 260) definiert, die bemessen und
eingerichtet ist, um die erste Wirbelsäulenstange darin zu empfangen, wobei die erste Passage (60; 260) eine Schlitzartige Konfiguration hat mit einer Länge, die sich entlang einer Querachse (T₂) erstreckt, die quer zur Rotationsachse (R) verläuft, wobei das zweite Stangenverbinderelement (22; 222) eine zweite Passage (40; 240) definiert, die bemessen und
eingerichtet ist, um darin die zweite Wirbelsäulenstange zu empfangen,
ein Verriegelungselement (26; 226), das wenigstens teilweise zwischen dem ersten und dem zweiten Stangenverbinderelement (24, 22; 224, 222) positioniert ist, wobei das Verriegelungselement (26; 226) aufweist eine abgewinkelte Stangenlagerfläche (84) und eine entgegensetzte erste Schnittstellenfläche (86), wobei die abgewinkelte Stangenlagerfläche sich entlang einer Ebene erstreckt, die in einem geneigten Winkel relativ zu der Rotationsachse (R) ausgerichtet, und
ein Kompressionselement (34), das sich in die erste Passage (60; 260) des ersten Stangenverbinderelements (24; 224) und in einen Presseingriff mit der ersten Wirbelsäulenstange erstreckt, um die erste Wirbelsäulenstange entlang der Querachse (T₂) und entlang der Länge des langgestreckten Schlitzes und in einen Gleiteingriff mit der abgewinkelten Stangenlagerfläche (84) des Verriegelungselements (26; 226) zu verlagern, um die erste Wirbelsäulenstange in der ersten Passage (60; 260) zu sichern, und
wobei der Gleiteingriff der ersten Wirbelsäulenstange gegen die Stangenlagerfläche (84) das Verriegelungselement (26; 226) axial zu dem zweiten Stangenverbinderelement (22; 222) im Allgemeinen entlang der Rotationsachse (R) verlagert, um die erste Schnittstellenfläche (86) des Verriegelungselements (26; 226) gegen eine gegenüberliegende zweite Schnittstellenfläche (46) zu pressen, um selektiv die Relativrotation zwischen dem ersten und dem zweiten Stangenverbinderelement (24, 22; 224, 222) zu verhindern,
wobei wenigstens eines von dem ersten und dem zweiten Stangenverbinderelement (24, 22; 224, 222) eine Lateralöffnung (41; 241) definiert, die mit einer korrespondierenden von der ersten und der zweiten Passage (60, 40; 260, 240) kommuniziert, um es einer von der ersten und der zweiten Wirbelsäulenstange (R1, R2) zu erlauben, lateral durch die Lateralöffnung (41; 241) und in die korrespondierende von der ersten und der zweiten Passage eingesetzt zu werden, und wobei die Rotationsachse (R) die Lateralöffnung (41; 241) schneidet.

2. Einstellbarer Verbinder (20; 200) gemäß Anspruch 1, wobei die Querachse (T₂) des langgestreckten Schlitzes in einem geneigten Winkel relativ zur Rotationschase (R) angeordnet ist.

3. Einstellbarer Verbinder (20; 200) gemäß Anspruch 2, wobei der langgestreckte Schlitz begrenzt ist von einander zugewandten und im Wesentlichen parallelen Seitenflächen, die sich entlang der Querachse (T₂)) im genannten geneigten Winkel erstrecken.

4. Einstellbarer Verbinder (20; 200) gemäß irgendeinem der Ansprüche 1-3, wobei die abgewinkelte Stangenlagerfläche (84) entlang der genannten Ebene im Wesentlichen flach und eben ist.

5. Einstellbarer Verbinder (20; 200) gemäß Anspruch 4, wobei die abgewinkelte Stangenlagerfläche (84) von einem oberen Abschnitt des Verriegelungselements (26; 226) aus über die Rotationsachse (R) hinweg und entlang einem unteren Abschnitt des Verriegelungselements im Wesentlichen flach und eben ist.

6. Einstellbarer Verbinder (20; 200) gemäß irgendeinem der Ansprüche 1-5, wobei die zweite Schnittstellenfläche (46) definiert ist durch eine Außenfläche des zweiten Stangenverbinderelements (22; 222).

7. Einstellbarer Verbinder (20; 200) gemäß Anspruch 6, wobei das Verriegelungselement (26; 226) aufweist ein Einstück-Verriegelungselement, das zwischen der ersten Wirbelsäulenstange (R₁; R₂), die innerhalb des langgestreckten Schlitzes in dem ersten Stangenverbinderelement (24; 224) positioniert ist, und der zweiten Schnittstellenfläche (46) angeordnet ist, die von der Außenfläche des zweiten Stangenverbinderelements (22; 222) definiert ist.

8. Einstellbarer Verbinder (20; 200) gemäß irgendeinem der Ansprüche 1-7, wobei die erste und die zweite Schnittstellenfläche (86) aufweisen eine Anzahl von sich radial erstreckenden Rippen im Eingriff mit einer Anzahl von sich radial erstreckenden Rillen, um selektiv die Relativrotation zwischen dem ersten und dem zweiten Stangenverbinderelement (24; 22; 224; 222) zu verhindern.

9. Einstellbarer Verbinder (20; 200) gemäß irgendeinem der Ansprüche 1-8, wobei das Kompressionselement aufweist ein erstes Kompressionselement (34), das sich in die erste Passage (60; 260) des ersten Stangenverbinderelements (24; 224) und in einen Presseingriff mit der ersten Wirbelsäulenstange erstreckt, um die erste Wirbelsäulenstange in der ersten Passage zu sichern, und
ferner aufweisend ein zweites Kompressionselement (32; 232), das sich in die Passage (40; 240) des zweiten Stangenverbinderelements (22; 222) und in einen Presseingriff mit der zweiten Wirbelsäulenstange erstreckt, um die zweite Wirbelsäulenstange in der zweiten Passage zu sichern.

10. Einstellbarer Verbinder (20; 200) gemäß Anspruch 9, ferner aufweisend ein Paar genannter erster Kompressionselemente, die sich in die erste Passage des ersten Stangenverbinderelements (24; 224) und in einen Presseingriff mit der ersten Wirbelsäulenstange erstrecken, um die erste Wirbelsäulenstange in der ersten Passage zu sichern, und
ein Paar zweiter Kompressionselemente, die sich in die zweite Passage des zweiten Stangenverbinderelements (22; 222) und in einen Presseingriff mit der zweiten Wirbelsäulenstange erstrecken, um die zweite Wirbelsäulenstange in der zweiten Passage zu sichern.

11. Einstellbarer Verbinder (20; 200) gemäß irgendeinem der Ansprüche 1-10, wobei eines von dem ersten und dem zweiten Stangenverbinderelement (24, 22; 224; 222) aufweist eine Öffnung (44), die sich im Allgemeinen entlang der Rotationsachse (R) erstreckt, und wobei das andere von dem ersten und dem zweiten Stangenverbinderelement aufweist einen Schaft (64), der mit der Öffnung in einem Dreheingriff ist, um die Relativrotation zwischen dem ersten und dem zweiten Stangenverbinderelement um die Rotationsachse (R) zu ermöglichen, wobei die Öffnung (44) aufweist einen Hinterschneidungsbereich (50), der eine Ringnut definiert, wobei der Schaft (64) aufweist einen vergrößerten Umfangsabschnitt, der sich davon ausgehend quer erstreckt und der in der Ringnut positioniert ist, um den Schaft (64) in der Öffnung (44) im Formschluss zu halten, um den Dreheingriff zwischen dem ersten und dem zweiten Stangenverbinderelement (24, 22; 224; 222) aufrechtzuhalten.

## Revendications

1. Connecteur ajustable (20; 200) pour interconnecter des première et seconde tiges vertébrales allongées (R₁ ; R₂) à des orientations angulaires sélectionnées l'une par rapport à l'autre, en combinaison avec lesdites tiges vertébrales allongées, comprenant :
un premier élément de connecteur de tige (24 ; 224) et un second élément de connecteur de tige (22 ; 222), dans lequel le premier élément de connecteur de tige (24 ; 224) est couplé en rotation au second élément de connecteur de tige (22 ; 222) pour permettre la rotation relative entre lesdits premier et second éléments de connecteur de tige autour d'un axe de rotation (R), ledit premier élément de connecteur de tige (24 ; 224) définissant un premier passage (60 ; 260) dimensionné et configuré pour y recevoir la première tige vertébrale, ledit premier passage (60 ; 260) a une configuration en forme de fente ayant une longueur s'étendant le long d'un axe transversal (T₂) agencé transversalement par rapport audit axe de rotation (R), ledit second élément de connecteur de tige (22 ; 222) définissant un second passage (40 ; 240) dimensionné et configuré pour y recevoir la seconde tige vertébrale ;
un élément de verrouillage (26 ; 226) positionné au moins partiellement entre lesdits premier et second éléments de connecteur de tige (24, 22 ; 224, 222), ledit élément de verrouillage (26 ; 220) comprenant une surface de support de tige coudée (84) et une première surface d'interface (86) opposée, ladite surface de support de tige coudée s'étendant le long d'un plan orienté à un angle oblique par rapport audit axe de rotation (R) ; et
un élément de compression (34) s'étendant dans ledit premier passage (60 ; 260) dudit premier élément de connecteur de tige (24 ; 224) et en mise en prise comprimée avec ladite première tige vertébrale pour déplacer ladite première tige vertébrale le long dudit axe transversal (T₂) et le long de ladite longueur de ladite fente allongée et en mise en prise coulissante avec ladite surface de support de tige coudée (84) dudit élément de verrouillage (26 ; 226) pour fixer ladite première tige vertébrale à l'intérieur dudit premier passage (60 ; 260) ; et
dans lequel la mise en prise coulissante de ladite première tige vertébrale contre ladite surface de support de tige (84) déplace axialement ledit élément de verrouillage (26 ; 226) vers ledit second élément de connecteur de tige (22 ; 222) généralement le long dudit axe de rotation (R) pour comprimer ladite première surface d'interface (86) dudit élément de verrouillage (26 ; 226) contre une seconde surface d'interface (46) opposée pour empêcher sélectivement ladite rotation relative entre lesdits premier et second éléments de connecteur de tige (24, 22 ; 224, 222),
dans lequel au moins l'un desdits premier et second éléments de connecteur de tige (24, 22 ; 224, 222) définit une ouverture latérale (41 ; 241) communiquant avec un passage correspondant desdits premier et second passages (60, 40 ; 260, 240) pour permettre à l'une desdites première et seconde tiges vertébrales (R₁, R₂) d'être latéralement insérée à travers ladite ouverture latérale (41 ; 241) et dans ledit passage correspondant desdits premier et second passages, et
dans lequel ledit axe de rotation (R) coupe ladite ouverture latérale (41 ; 241).

2. Connecteur ajustable (20 ; 200) selon la revendication 1, dans lequel ledit axe transversal (T₂) de ladite fente allongée est agencé à un angle oblique par rapport audit axe de rotation (R).

3. Connecteur ajustable (20 ; 200) selon la revendication 2, dans lequel ladite fente allongée est délimitée par des surfaces latérales orientées à l'opposé et sensiblement parallèles s'étendant le long dudit axe transversal (T₂) audit angle oblique.

4. Connecteur ajustable (20 ; 200) selon l'une quelconque des revendications 1 à 3, dans lequel ladite surface de support de tige coudée (84) est sensiblement plate et plane le long dudit plan.

5. Connecteur ajustable (20 ; 200) selon la revendication 4, dans lequel ladite surface de support de tige coudée (84) est sensiblement plate et plane à partir d'une partie supérieure dudit élément de verrouillage (26 ; 226) sur ledit axe de rotation (R) et le long d'une partie inférieure dudit élément de verrouillage.

6. Connecteur ajustable (20 ; 200) selon l'une quelconque des revendications 1 à 5, dans lequel ladite seconde surface d'interface (46) est définie par une face externe dudit second élément de connecteur de tige (22 ; 222).

7. Connecteur ajustable (20 ; 200) selon la revendication 6, dans lequel ledit élément de verrouillage (26 ; 226) comprend un élément de verrouillage monobloc positionné entre ladite première tige vertébrale (R₁ ; R₂) positionnée à l'intérieur de ladite fente allongée dans ledit premier élément de connecteur de tige (24 ; 224) et ladite seconde surface d'interface (46) définie par ladite face externe dudit second élément de connecteur de tige (22 ; 222).

8. Connecteur ajustable (20 ; 200) selon l'une quelconque des revendications 1 à 7, dans lequel lesdites première et seconde surfaces d'interface (86 ; 46) comprennent un certain nombre de cannelures s'étendant de manière radiale, s'entrelaçant avec un certain nombre de rainures s'étendant de manière radiale pour empêcher sélectivement ladite rotation relative entre lesdits premier et second éléments de connecteur de tige (24, 22 ; 224 ; 222).

9. Connecteur ajustable (20 ; 200) selon l'une quelconque des revendications 1 à 8, dans lequel ledit élément de compression comprend un premier élément de compression (34) s'étendant dans ledit premier passage (60 ; 260) dudit premier élément de connecteur de tige (24 ; 224) et en mise en prise comprimée avec ladite première tige vertébrale pour fixer ladite première tige vertébrale à l'intérieur dudit premier passage ; et
comprenant en outre un second élément de compression (32 ; 232) s'étendant dans ledit second passage (40 ; 240) dudit second élément de connecteur de tige (22 ; 222) et en mise en prise comprimée avec ladite seconde tige vertébrale pour fixer ladite seconde tige vertébrale à l'intérieur dudit second passage.

10. Connecteur ajustable (20 ; 200) selon la revendication 9, comprenant en outre une paire desdits premiers éléments de compression s'étendant dans ledit premier passage dudit premier élément de connecteur de tige (24 ; 224) et en mise en prise comprimée avec ladite première tige vertébrale pour fixer ladite première tige vertébrale à l'intérieur dudit premier passage ; et
une paire desdits seconds éléments de compression s'étendant dans ledit second passage dudit second élément de connecteur de tige (22 ; 222) et en mise en prise comprimée avec ladite seconde tige vertébrale pour fixer ladite seconde tige vertébrale à l'intérieur dudit second passage.

11. Connecteur ajustable (20 ; 200) selon l'une quelconque des revendications 1 à 10, dans lequel l'un desdits premier et second éléments de connecteur de tige (24, 22 ; 224 ; 222) comprend une ouverture (44) s'étendant généralement le long dudit axe de rotation (R) et dans lequel l'autre desdits premier et second éléments de connecteur de tige comprend un arbre (64) mis en prise en rotation à l'intérieur de ladite ouverture pour permettre ladite rotation relative entre lesdits premier et second éléments de connecteur de tige autour dudit axe de rotation (R), ladite ouverture (44) comprenant une région de dégagement (50) définissant une rainure annulaire, ledit arbre (64) comprenant une partie périphérique agrandie s'étendant transversalement à partir de ce dernier et étant positionnée à l'intérieur de ladite rainure annulaire pour capturer positivement ledit arbre (64) à l'intérieur de ladite ouverture (44) pour maintenir la mise en prise rotative entre lesdits premier et second éléments de connecteur de tige (24, 22 ; 224 ; 222).
